(19) Europäisches Patentamt
European Patent Office
Office européen des brevets

(11) **EP 3 632 245 B1**

(12) **EUROPEAN PATENT SPECIFICATION**

(45) Date of publication and mention
of the grant of the patent:
**15.09.2021 Bulletin 2021/37**

(51) Int Cl.:
*A41B 11/00* *(2006.01)* *A61F 13/08* *(2006.01)*

(21) Application number: **17912277.5**

(86) International application number:
**PCT/JP2017/020324**

(22) Date of filing: **31.05.2017**

(87) International publication number:
**WO 2018/220764 (06.12.2018 Gazette 2018/49)**

(54) **COMPRESSION STOCKING**

KOMPRESSIONSSTRUMPF

BAS DE CONTENTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**08.04.2020 Bulletin 2020/15**

(73) Proprietor: **Asahi Kasei Kabushiki Kaisha
Tokyo 100-0006 (JP)**

(72) Inventors:
• **DEGUCHI, Junko
Tokyo 100-0006 (JP)**
• **SAEGUSA, Narumi
Tokyo 100-0006 (JP)**
• **AKITA, Shoichi
Tokyo 100-0006 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(56) References cited:
WO-A1-2010/082677      JP-A- H11 131 303
JP-A- 2000 290 806       JP-A- 2007 239 151
JP-A- 2008 543 404       JP-A- 2011 515 173
JP-A- 2017 095 838       US-A1- 2003 213 269
US-A1- 2012 116 282

• SERGE COUZAN ET AL: "A randomized
double-blind trial of upward progressive versus
degressive compressive stockings in patients
with moderate to severe chronic venous
insufficiency", JOURNAL OF VASCULAR
SURGERY, vol. 56, no. 5, 1 November 2012
(2012-11-01), pages 1344-1350.e1, XP055689231,
AMSTERDAM, NL ISSN: 0741-5214, DOI:
10.1016/j.jvs.2012.02.060

EP 3 632 245 B1

## Description

FIELD

The present invention relates to a compression stocking. More particularly, the present invention relates to a compression stocking having a swelling reduction effect while demonstrating superior removability by enhancing compression of a calf portion together with increasing elongation of an ankle portion in the radial direction and reducing initial tensile stress of the ankle portion in the radial direction.

BACKGROUND

Hosiery and stockings have been typically known in the past that have a "graduated compression design" that causes tightening pressure to change from strong to weak moving upward from the periphery of the lower limbs for the purpose of alleviating swelling and fatigue of the lower limbs. According to this design, the ankle portion is typically subjected to particularly high compression since it is necessary to apply compression to the periphery, and the ankle portion has typically been made to be resistant to elongation in the radial direction in order to enhance compression. Moreover, the majority of compression stockings easily stretch in the lengthwise direction, and products having such stretching characteristics require considerable force to put on and take off, thereby creating a sense of inconvenience for patients with venous diseases, and particularly women and the elderly having reduced arm strength. Although one example thereof is a stocking that finely regulates compression by employing a "graduated compression design" for the above-mentioned purpose as disclosed in the following PTL1, although this stocking takes into consideration the sense of fit, it has the problem of not considering removability.
In addition, as is disclosed in the following PTL2, although a lower limb article of clothing for engaging in sports is disclosed that imparts pressure to a portion covering the ankle and calf and lowers the elongation rate in the lengthwise direction mores than the elongation rate in the radial direction of a calf portion to make it more difficult to stretch in the lengthwise direction, there is no description of lowering elongation rate in the lengthwise direction of the calf portion as a method for facilitating removability. Since elongation rate of the ankle portion is more important than that of the calf portion in order to facilitate removability, it is difficult to obtain favorable removability simply by adjusting the elongation rate of the calf portion. US 2003/0213269 A1 discloses a therapeutic stocking knitted with a jersey knit structure on a conventional circular knit hosiery machine. The leg yarn is nylon and the inlay yarn is bare spandex. The leg portion of the stocking exerts a compressive pressure from 18 mm Hg at the ankle and gradually decreases to 8 mm Hg at the upper thigh.

[CITATION LIST]

[PATENT LITERATURE]

[PTL 1] Japanese Unexamined Patent Publication No. 2005-240222
[PTL 2] Japanese Unexamined Patent Publication No. 2010-77574

SUMMARY

[TECHNICAL PROBLEM]

With the foregoing in view, an object of the present invention is to provide a compression stocking having favorable removability while alleviating swelling and fatigue of the lower limbs.

[SOLUTION TO PROBLEM]

The inventors of the present invention conducted extensive studies to solve the aforementioned problems, achieved knitted fabric stretching characteristics important for removability, and repeated experiments such as wear tests, the results of which led to completion of the present invention.
Namely, the present invention is as described below.

[1] A compression stocking that at least covers an ankle portion, a lower calf portion and a calf portion, wherein stress when the calf portion is stretched in the radial direction to a worn state is 1 N to 7 N, stress when the ankle portion is stretched in the radial direction until it passes over the heel is 1 N to 7 N, and initial load when the ankle portion is stretched in the radial direction to an elongation rate of 10% is 1 N or less, characterized in that the elongation rate of the ankle portion (b) in the lengthwise direction during a load of 22.1 N is 10 % to 100 %.

[2] The compression stocking described in [1] above, wherein the compression stocking is composed by circular knitting.

[3] The compression stocking described in [1] or [2] above, wherein elastic yarn is insert-knitted into a portion at least containing the ankle portion, lower calf portion and calf portion of the compression stocking.

[4] The compression stocking described in [3] above, wherein the elastic yarn is insert-knitted by using bare yarn.

[5] The compression stocking described in any of [1] to [4] above, wherein compression of the calf portion is 15 hPa to 50 hPa.

[6] The compression stocking described in [5] above, wherein compression of the lower calf portion is 3 hPa to 15 hPa greater than the compression of the calf portion.

[7] The compression stocking described in [5] or [6], wherein compression of an instep portion is 3 hPa to 15 hPa greater than the compression of the calf portion.

[8] The compression stocking described in any of [1] to [7] above, wherein elongation rate of the ankle portion in the lengthwise direction during a load of 22.1 N is 10% to 70%.

[9] The compression stocking described in any of [1] to [8] above, wherein the standard deviation of the dynamic friction coefficient on the skin side of the ankle portion is 0.005 to 0.08.

[10] The compression stocking described in any of [5] to [9] above, wherein compression of the ankle portion is 5 hPa to 35 hPa and smaller than the compression of the calf portion.

[11] The compression stocking described in any of [1] to [10], wherein air permeability of the calf portion is 150 cc/cm$^2$·s or more.

[12] The compression stocking described in any of [1] to [11] above, wherein the moisture absorption rate is 7% or more.

[ADVANTAGEOUS EFFECTS OF INVENTION]

The compression stocking of the present invention applies pressure to a calf portion, the ankle portion of the stocking having the smallest circumference easily stretches in the radial direction when passing over the heel when the stocking is put on and taken off, and initial tensile stress of the ankle portion in the radial direction is small, thereby resulting in superior removability while also having a swelling reduction effect.

BRIEF DESCRIPTION OF DRAWINGS

FIG. 1 is a model diagram of a lower limb corresponding to the sites of a compression stocking.
FIG. 2 is an example of a knitted fabric structural diagram used for a compression stocking.
FIG. 3 is an example of a knitted fabric structural diagram used for a compression stocking of the present embodiment.
FIG. 4 is an example of a knitted fabric structural diagram used for a compression stocking of the present embodiment.
FIG. 5 is an example of a conventional knitted fabric structural diagram (Comparative Example 1).
FIG. 6 is an example of a conventional knitted fabric structural diagram (Comparative Example 2).
FIG. 7 is a knitted fabric structural diagram in which it is difficult for elastic yarn to appear on the skin side surface in the same manner as FIG. 3.

DESCRIPTION OF EMBODIMENTS

The following provides a detailed explanation of embodiments of the present invention.

The compression stocking of the present embodiment is a compression stocking that at least covers an ankle portion, a lower calf portion and a calf portion, wherein stress when the calf portion is stretched in the radial direction to a worn state is 1 N to 7 N, stress when the ankle portion is stretched in the radial direction until it passes over the heel is 1 N to 7 N, and initial load when the ankle portion is stretched in the radial direction to an elongation rate of 10% is 1 N or less, characterized in that the elongation rate of the ankle portion (b) in the lengthwise direction during a load of 22.1 N is 10 % to 100 %. Examples of specific embodiments include a high sock type of a length extending from the toes to below or above the knee, a gator type of a length extending from the ankle to below the knee, and a panty stocking type extending from the toes to the waist.

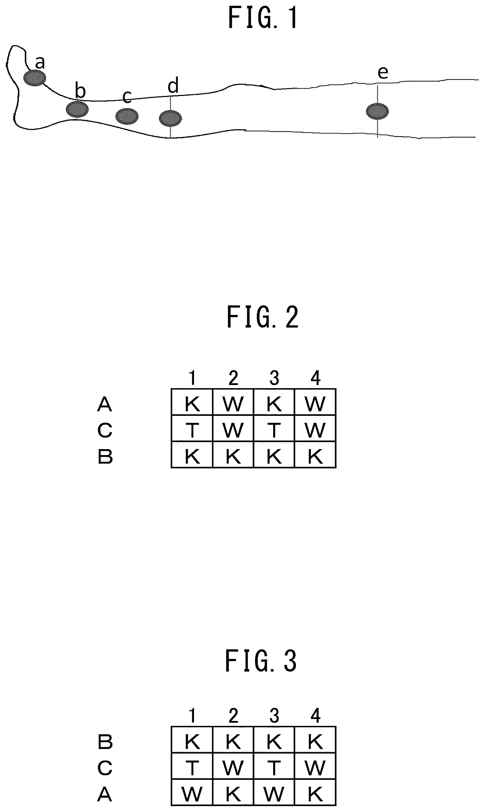

The instep portion, ankle portion, lower calf portion, calf portion and thigh portion of a compression stocking refer to the sites indicated in FIG. 1. The instep portion is indicated by (a) in FIG. 1 and refers to the site that includes an intermediate point between the base of the foot and base of the toes. The ankle portion is indicated by (b) in FIG. 1 and refers to the site that includes the region having the smallest circumference over the ankle bone of the ankle. The calf portion is indicated by (d) in FIG. 1 and refers to the site that includes the region of the calf having the largest circumference. The lower calf portion is indicated by (c) in FIG. 1 and refers to the intermediate site between (b) and (d). The thigh portion is indicated by (e) in FIG. 1 and refers to site intermediate to the knee and the base of the leg.

The compression stocking of the present embodiment is characterized in that stress when the calf portion is stretched in the radial direction to a worn state is 1 N to 7 N. This stress is preferably 2.5 N to 4.5 N. Being stretched to a worn state refers to stretching to the circumference (cm) of the calf portion of a wooden model leg for measuring compression manufactured by Swiss Lastic. More specifically, since a size M stocking, for example, corresponds to a calf circumference of the human body of 32 cm to 38 and a type 7 is used for the model leg, the circumference of the calf portion of this model leg is 34.5 cm. Since a size L stocking, for example, corresponds to a calf circumference of the human body of 36 cm to 42 cm and a type 10 is used for the model leg, the circumference of the calf portion of the model leg is 39 cm. Since a size S stocking, for example, corresponds to a calf circumference of the human body of 28 cm to 34 cm and a type 5 is used for the model leg, the circumference of the calf portion of the model leg is 30.5 cm. The compression stocking of the present embodiment enables suitable pressure to be applied to the calf portion since stress when the calf portion is stretched in the radial direction to the worn state is 1 N to 7 N. If this stress is less than 1 N, compression is excessively low making it difficult to obtain a swelling preventive effect. On the other hand, if this stress exceeds 7 N, stress becomes excessively high making it difficult to put on the stocking and causing congestion, thereby making this undesirable.

Furthermore, in the case of a compression stocking of unknown size, the size is considered to be size M if stress measured when the calf portion is stretched in the radial direction to the worn state is 1 N to 7 N based on the assumption that the stocking is size M. If the stress is less than 1 N, the stocking is considered to be size S and if the stress is greater than 7 N, the stocking is considered to be size L.

The compression stocking of the present embodiment is characterized in that stress when the ankle portion is stretched in the radial direction until it passes over the heel is 1 N to 7 N. This stress is preferably 2.5 N to 4.5 N. In the case of conventional compression stockings, considerable pressure was normally applied to the ankle portion thereby making it difficult to put on the stocking while applying pressure.

Stretching until the stocking passes over the heel refers to stretching to the circumference (cm) of the heel of the aforementioned wooden model leg for measuring compression manufactured by Swiss Lastic corresponding to size S, M or L. For example, the stocking is stretched to a circumference of 29 cm in the case of a type 5 model leg, 31 cm in the case of a type 7 model leg, and 34 cm in the case of type 10 model leg. The compression stocking is stretched the most in the radial direction when worn when an ankle portion having a small circumference is passed over the heel portion. Thus, ease of wearing the stocking is improved by reducing the stress at this time. If the stress is less than 1 N, the circumference of the ankle prior to wearing the compression stocking ends up becoming excessively large and the appearance is not preferable. On the other hand, if the stress exceeds 7 N, the amount of stress required when putting the stocking on increases and the stocking becomes difficult to put on, thereby making this undesirable.

The compression stocking of the present embodiment is characterized in that the initial load when the ankle portion is stretched in the radial direction to an elongation rate of 10% is 1 N or less. If initial stress of the ankle portion having the smallest circumference is large when the stocking is worn, force is required when placing the toes in the stocking, thereby making the stocking extremely difficult to put on. The toes can be easily placed in the stocking thereby improving the ease of wearing by making the initial load to be 1 N or less.

In addition, to reduce the elongation rate in the lengthwise direction of the stocking in order to improve ease of wearing, the elongation rate of the ankle portion in the lengthwise direction during a load of 22.1 N is 10% to 100% and preferably 30% to 80%. If the elongation rate of the ankle portion in the lengthwise direction during a load of 22.1 N is less than 10%, the stocking is unable to match stretching of the skin during movement of the body, thereby causing the stocking to slip down or cause a sense of tightness during movement while wearing the stocking. On the other hand, if the elongation rate of the ankle portion in the lengthwise direction during a load of 22.1 N exceeds 100%, force that causes the stocking to widen when putting on and taking off the stocking may be easily used for elongation in the lengthwise direction or force greater than or equal to that required may be required to be applied to widen the ankle portion in the radial direction, thereby making it difficult to put on and take off the stocking. Removability is favorable by making the elongation rate of the ankle portion in the radial direction during a load of 22.1 N to be within this range.

In the compression stocking of the present embodiment, compression of the calf portion is preferably 15 hPa to 50 hPa, more preferably 20 hPa to 40 hPa and even more preferably 20 hPa to 35 hPa. As a result of making compression of the calf portion to be a prescribed pressure, venous return is promoted, and that effect is remarkable in the case compression of the calf portion is 15 hPa to 50 hPa. Whether or not venous return is promoted can be evaluated by measuring swelling after wearing for one day. Swelling can be evaluated according to the amount of change in circumference. Venous blood of the lower limbs is required to flow in the opposite direction of body weight when assuming a posture such as sitting or standing and results in increased susceptibility to backflow in cases of remaining in a standing position or in cases in which muscle pumping action is difficult to act such as in cases of weak muscles in women or the elderly. Application of a prescribed pressure to the calf portion makes it possible to prevent venous dilation, and together with reducing backflow of venous blood, makes it possible to expect an action that reduces blood retention by lower limb veins, thereby making it possible to expect not only suppression of swelling of the lower limbs, but also suppression of the formation of varicose veins and expectation of an effect that suppresses thrombus formation.

Since pressure is low at a compression of the calf portion of less than 15 hPa, the effect of preventing venous dilation is small and the effect of promoting venous return ends up diminishing. On the other hand, if compression of the calf portion exceeds 50 hPa, pressure becomes excessively strong resulting in a sense of stiffness, veins being crushed and the risk of inhibiting circulation.

As a result of measuring swelling after wearing compression stockings designed to exhibit various pressures, the inventors of the present invention found that an even greater effect of promoting venous return is observed by compressing the lower calf portion. In the compression stocking of the present embodiment, the lower calf portion is preferably compressed at 3 hPa to 15 hPa greater than compression of the calf portion and more preferably 3 hPa to 10 hPa greater than compression of the central calf portion.

Since compression of the instep portion also contributes to promotion of venous return, in the compression stocking of the present embodiment, compression of the instep portion is preferably designed to be 3 hPa to 15 hPa greater than compression of the calf portion and more preferably 3 hPa to 10 hPa greater than compression of the calf portion.

Improvement of swelling can be expected as a result of pushing on pressure points since pressure is applied to the lower calf portion and instep portion.

On the other hand, in the case of a design that enhances compression of the ankle as was commonplace in the past, significant swelling preventive effects were unable to be obtained in an evaluation of swelling using compression stockings designed to exhibit various pressures conducted by inventors of the present invention. When compression of the ankle is enhanced, compression of the portion having an extremely small circumference is enhanced making the stocking extremely difficult to put on. Even in the case of employing a knitting structure that increases horizontal elongation of the ankle portion in the manner of the compression stocking of the present embodiment, there ends up being limitations on the ease with which the stocking can be put on in the case compression of the ankle portion is excessively high. Thus, compression of the ankle portion is preferably small relative to the calf portion. In the compression stocking of the present embodiment, compression of the ankle portion is preferably 10 hPa to 35 hPa and more preferably 12 hPa to 30 hPa. If compression of the ankle portion is less than 10 hPa, there is the risk of the stocking slipping when worn. On the other hand, if compression of the ankle portion exceeds 35 hPa, ease of wearing may be insufficient even if a suitable knitting structure is employed.

Even in the case of employing a graduated compression design, which has conventionally considered to be important in order to demonstrate the effect of promoting venous return, it is preferable to increase compression of the ankle as little as possible.

It is preferable to apply a graduated compression design by enhancing compression of either or both the lower calf portion and instep portion relative to the central calf portion.

As a result, the venous return effect is large while still employing a graduated compression design, thereby making it possible to achieve a compression stocking that is extremely easy to put on.

In the case the compression stocking of the present embodiment includes a thigh portion, compression of the thigh portion is preferably 5 hPa to 30 hPa less than that of the calf portion. If a strong pressure is applied to the thigh portion, ease of wearing is inhibited and a sense of stiffness when wearing the stocking becomes extremely strong.

It is preferable to adopt the form of a circular knit in order to achieve this compression design and stretching characteristics, a panty stocking knitting machine for high compression is used preferably and the knitted fabric is preferably composed by combining non-elastic yarn and elastic yarn.

An elastic yarn is preferably insert-knitted in order to achieve this compression design and stretching characteristics. If a non-elastic yarn is plate stitched instead of insert knitting an elastic yarn, the compression stocking easily stretches in the lengthwise direction making it difficult to put on and take off the stocking. As a result insert knitting an elastic yarn, extensibility of the elastic yarn in the radial direction of the compression stocking is demonstrated in particular, and since the power of the elastic yarn directly leads to the magnitude of compression, control of compression becomes easy.

Polyurethane yarn, polyether-ester elastic yarn, polyamide elastic yarn, polyolefin elastic yarn or these yarns covered with a non-elastic yarn or in a covered state may be used for the elastic yarn used in the compression stocking of the present embodiment. Although so-called rubber yarn, which is a form of yarn comprised of natural rubber, synthetic rubber or semisynthetic rubber, can also be used, polyurethane elastic yarn is preferably due to its superior elasticity and common use. There are no particular limitations on the polyurethane structure, the structure may be that of polyurethane having a polyester structure or polyurethane having a polyether structure, and among these, a structure having a side chain in at least a portion of the polyether skeleton as a diol component is preferable. A side chain having a methyl group is preferable for the side chain and that having two of the aforementioned methyl groups on the same carbon is particularly preferable.

The polyurethane elastic yarn is preferably 50 dtex to 500 dtex and is used in the form of bare yarn, and more preferably is 100 dtex to 350 dtex elastic yarn. Bare yarn has superior stretchability since there is no impairment of other yarn during stretching as compared with covered yarn. In addition, since bare yarn is narrower in comparison with covered yarn, there is less susceptibility to the occurrence of surface irregularities attributable to differences in yarn thickness on the surface of the knitted fabric, thereby resulting in a smooth touch to the skin and favorable air permeability.

In the case of using a covered yarn, a narrow yarn of 8 dtex to 56 dtex and preferably 8 dtex to 33 dtex is used and the number of windings is preferably 500 times/m or less.

The non-elastic yarn used in the compression stocking of the present embodiment preferably composes a loop separate from the elastic yarn, and may be a non-elastic yarn having a yarn insert structure other than the non-elastic yarn that composes the loop. The non-elastic yarn may be a filament yarn or spun yarn. More specifically, filament yarn comprised of chemical weaving of fibers such as polyamide-based fiber, polyester-based fiber, acrylic-based fiber, polypropylene-based fiber or vinyl chloride-based fiber is preferable for the filament yarn. In addition, cellulose fiber such as cupra, rayon, bamboo fiber, cotton, modal or tencel is also preferable, and regenerated cellulose fiber is particularly preferable. In addition, the filament yarn may also be a composite yarn with, for example, polyester-based fiber or polyamide-based fiber. In particular, a composite yarn such as that composed of cellulose fiber and polyester-based fiber or polyamide-based fiber is more preferable as a result of being able to impart strength, durability and moisture absorption to the compression stocking.

The composite yarn may be air blended, covered or false twist blended and the like, and there are no particular limitations thereon. The cross-sectional shape of the filament yarn may be circular (O), triangular (△), cross-shaped, W-shaped, M-shaped, C-shaped, I-shaped, dog bone-shaped or hollow and the like, and there are no particular limitations thereon. Spun yarn comprised of natural fiber such as cotton, wool or hemp, regenerated fiber such as cupra, rayon or bamboo fiber, or chemically woven fiber such as polyamide-based fiber, polyester-based fiber, acrylic-based fiber, polypropylene-based fiber or vinyl chloride fiber is preferable for the spun yarn and these may consist of a single fiber or blend. Although there are no particular limitations on the blending method, spun yarn obtained using a Murata Vortex Spinner (MVS) that is resistant to pilling is preferable. In other words, a suitably preferable material is selected according to the application.

Fineness of the non-elastic yarn in the case of filament yarn is preferably 50 dtex to 240 dtex and more preferably 60 dtex to 170 dtex. Since elastic yarn is stretched by a factor of 1.2 times to 2.5 times when worn, differences in fineness with the elastic yarn can be eliminated by making the fineness of the non-elastic yarn to be 1/1.2 to 1/2.5 the fineness of the elastic yarn, thereby resulting in a compression stocking that is smooth, easy to put on, and has a favorable texture on the skin. In the case of spun yarn, the spun yarn preferably has a count of 60 to 20 and more preferably a count of 50 to 30.

Single yarn fineness is preferably 0.5 dtex to 8 dtex and more preferably 1 dtex to 6 dtex. In the case single yarn fineness is less than 0.5 dtex, yarn strength becomes insufficient and the yarn breaks easily. If the single yarn fineness exceeds 8 dtex, there is the risk of the yarn becoming hard and straw-like and having poor texture on the skin.

In the compression stocking of the present embodiment, it is necessary to select a suitable knitting structure. Examples of knitting structures preferable for the present embodiment are shown in FIGS. 2 to 4 and FIG. 7. In these drawings, K stands for knit, T stands for tuck and W stands for welt. In addition, in each of the knitting structures, C represents elastic yarn while A, B and D represent non-elastic yarn. Since the elastic yarns of FIGS. 2 to 4 and FIG. 7 are all insert-knitted, the resulting structure facilitates elongation in the radial direction. In addition, elongation in the lengthwise direction is suppressed since the elastic yarns do not form loops, or in other words, are not connected.

In addition, loops can be inserted in the gaps of the knitted fabric to reduce surface irregularities in the knitted fabric by combining yarn having knit loops in which knit loops of non-elastic yarn catch on the second or third previous loop thereto and yarn in which knit loops of non-elastic yarn catch on all of the loops one loop previous thereto in the radial direction, thereby making this preferable.

Here, catching a knit loop of non-elastic yarn on the loop one loop previous thereto refers to knit loops of non-elastic yarn being continuous in the lengthwise direction (vertical direction in the drawings, lengthwise direction in the stocking) as is seen in columns 1 and 3 of FIG. 2 and columns 2 and 4 of FIG. 3 (elastic yarn is ignored since it is insert-knit and this applies similarly hereinafter). Catching a knit loop of non-elastic yarn on the second previous loop thereto refers to the knit loop of non-elastic yarn being two loops back in the lengthwise direction as is seen in columns 2 and 4 of FIG. 2 and columns 1 and 3 of FIG. 3.

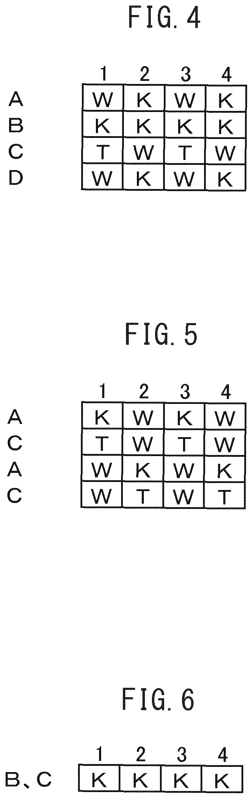

In the structures of FIGS. 2, 3 and 7, yarn having knit loops in which knit loops of non-elastic yarn catch on the second loop previous thereto (B) and yarn in which knit loops of non-elastic yarn catch on all of the loops one loop previous thereto (A) are arranged in the radial direction. In the structure of FIG. 4, yarn having knit loops in which knit loops of non-elastic yarn catch on the third loop previous thereto (B) and yarn in which knit loops of non-elastic yarn catch on all of the loops one loop previous thereto (A and D) are arranged in the radial direction.

As was previously described, in the compression stocking of the present embodiment, compression of the calf portion is preferably 15 hPa to 50 hPa, more preferably 20 hPa to 40 hPa and even more preferably 20 hPa to 35 hPa. Selection of the elastic yarn and optimization of yarn length are particularly important in each structure in order to control the compression of each portion. For example, in the case of the knitting structure shown in FIG. 2, in the case of using 235 dtex polyurethane yarn as elastic yarn for C in FIG. 2 and using 36 dtex nylon and 66 dtex cupra as non-elastic yarn for A and B in FIG. 2, the length of the non-elastic yarn of the calf portion is preferably 5.0 cm/100 wales to 7.0 cm/100 wales. In the knitting structure of FIG. 3, in the case of using 235 dtex polyurethane yarn as elastic yarn for C in FIG. 3 and using 36dtex nylon and 66 dtex cupra as non-elastic yarn for A and B in FIG. 3, the length of the non-elastic yarn of the calf portion is preferably 5.2 cm/100 wales and 7.2 cm/100 wales.

It is particularly necessary to use an appropriate structure and yarn length for the non-elastic yarn in order to ensure ease of elongation in the radial direction of the ankle portion. For example, in the case of the knitting structure of FIG. 2, in the case of using 235 dtex polyurethane yarn as elastic yarn for C in FIG. 2 and using 36 dtex nylon and 66 dtex cupra as non-elastic yarn for A and B in FIG. 2, the length ratio between the non-elastic yarn and elastic yarn on the all-knit side of the ankle portion is preferably 4.5 to 6.5 and more preferably 5.0 to 6.0. In addition, the length ratio between the non-elastic yarn and elastic yarn on the knit welt side is preferably 3.0 to 4.0 and more preferably 3.2 to 3.8.

If the yarn length ratio exceeds this range, non-elastic yarn rises to the surface resulting in poor smoothness. In addition, if the yarn length ratio is smaller than this range, the non-elastic yarn becomes excessively short, tightness occurs and removability is inferior.

The yarn length of the ankle portion is preferably such that the yarn length of size M non-elastic yarn on the all-knit side is preferably 22 cm/100 wales to 28 cm/100 wales and preferably 12 cm/100 wales to 18 cm/100 wales on the knit welt side. If the length of the non-elastic yarn is smaller than the above, removability may become poor since elongation decreases in the radial direction and elongational stress increases. In addition, if the non-elastic yarn exceeds the aforementioned yarn length, although elongation increases, non-elastic yarn ends up rising to the surface resulting in an increase in the dynamic friction coefficient and poor smoothness. Moreover, snagging may become poor. Yarn length of the elastic yarn is preferably 4 cm/100 wales to 5 cm/100 wales and more preferably 4.2 cm/100 wales to 4.8 cm/100 wales.

If yarn length of the elastic yarn is less than 4 cm/100 wales, elongational stress in the radial direction of the ankle portion increases and it becomes difficult to put on and take off the stocking. On the other hand, if yarn length of the elastic yarn exceeds 5 cm/100 wales, although removability improves, compression may be unable to be obtained that demonstrates the effect of alleviating swelling and fatigue.

In addition, in the knitting structure of FIG. 3, in the case of using 235 dtex polyurethane yarn as elastic yarn for C in FIG. 3 and using 36 dtex nylon and 66 dtex cupra as non-elastic yarn for A and B in FIG. 3, the yarn length ratio between the non-elastic yarn and elastic yarn on the all-knit side of the ankle portion is preferably 4.0 to 5.8 and more preferably 4.2 to 5.6. In addition, the yarn length ratio between the non-elastic yarn and elastic yarn on the knit welt side is preferably 3.5 to 4.5 and more preferably 3.7 to 4.3. The length of the non-elastic yarn of the ankle portion on the all knit side is preferably 19 cm/100 wales to 25 cm/100 wales and preferably 14 cm/100 wales to 20 cm/100 wales on the knit welt side. The length of the elastic yarn is preferably 4 cm/100 wales to 5 cm/100 wales and more preferably 4.2 cm/100 wales to 4.8 cm/100 wales.

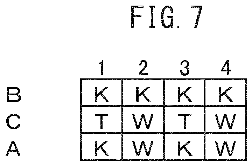

In FIG. 2, although the yarn is arranged in the lengthwise direction of the knitted fabric in the order of yarn having knit loops in which knit loops of the non-elastic yarn catch on the second loop previous thereto (B), elastic yarn (C), and yarn in which knit loops of the non-elastic yarn catch on all loops one loop previous thereto (A), in FIGS. 3 and 7, the yarn is arranged in the lengthwise direction of the knitted fabric in the order of yarn in which knit loops of the non-elastic yarn catch on all loops one loop previous thereto (A), elastic yarn (C), and yarn in which knit loops of the non-elastic yarn catch on the second loop previous thereto (B). Since the knitting structure of FIG. 3 results in a structure that makes it difficult for elastic yarn to appear on the skin side surface in comparison with the knitting structure of FIG. 2, smoothness is favorable and ease of putting on is superior. As a result of calculating the exposure rate of elastic yarn on the skin side surface of the calf portion in the state of being stretched while being worn from an enlarged photograph magnified by 100X with a microscope, the exposure rate of the structure of FIG. 2 was about 27% while that of the structure of FIG. 3 was about 18%.

Since the knitting structure of FIG. 7 also employs a structure in which it is difficult for elastic yarn to appear on the skin side surface in the same manner as that of FIG. 3, ease of putting on is superior and the effect of reducing glare is large. The surface exposure rate of elastic yarn of the knitting structure of FIG. 7 was about 19%.

The standard deviation of the dynamic friction coefficient on the skin side of the compression stocking of the present embodiment is preferably 0.005 to 0.08, more preferably 0.005 to 0.05 and even more preferably 0.005 to 0.03. If the standard deviation of the dynamic friction coefficient is less than 0.005, slipping over the skin occurs causing the stocking to easily shift out of position. On the other hand, if the standard deviation of the dynamic friction coefficient exceeds 0.08, frictional resistance with the skin increases when putting on and taking off the stocking, thereby making it difficult to put on and take off the stocking.

The standard deviation of the dynamic friction coefficient is preferably set to the range of 0.005 to 0.08 by employing a knitting structure like that shown in FIGS. 2 to 4, As a result of making knit loops of non-elastic yarn in the lengthwise direction at those locations where the elastic yarn forms tuck loops between the non-elastic yarn and elastic yarn in the lengthwise direction to be continuous with knit loops, the spaces between elastic yarn and elastic yarn are filled with knit loops of non-elastic yarn, thereby reducing surface irregularities and making it possible to minimize the standard deviation of the dynamic friction coefficient.

The dynamic friction coefficient on the skin side of the compression stocking of the present embodiment is preferably 0.1 to 0.5, more preferably 0.15 to 0.4 and even more preferably 0.15 to 0.3. If the dynamic friction coefficient is less than 0.1, slipping over the skin occurs causing the stocking to easily shift out of position. On the other hand, if the dynamic friction coefficient exceeds 0.5, frictional resistance with the skin increases when putting on and taking off the stocking, thereby making it difficult to put on and take off the stocking.

Surface roughness of the skin side of the compression stocking of the present embodiment is preferably 1 $\mu$m to 10 $\mu$m, more preferably 3 $\mu$m to 8 $\mu$m and even more preferably 3 $\mu$m to 6 $\mu$m. If surface roughness is less than 1 $\mu$m, slipping over the skin occurs causing the stocking to easily shift out of position. On the other hand, if surface roughness exceeds 10 $\mu$m, the compression stocking catches on the skin when putting on and taking off, thereby making it difficult to put on and take off the stocking. Texture on the skin also feels rough, thereby making this undesirable.

The thickness of the compression stocking of the present embodiment is preferably 0.4 mm to 0.9 mm and more preferably 0.4 mm to 0.8 mm. If the thickness is less than 0.4 mm, the sense of sheerness becomes extremely strong while also resulting in susceptibility to tearing when putting on and taking off. On the other hand, if the thickness exceeds 0.9 mm, the stocking feels hot and sticky.

Air permeability of the compression stocking of the present embodiment is preferably 300 cc/cm$^2\cdot$s or more, more preferably 600 cc/cm$^2\cdot$s or more and even more preferably 1000 cc/cm$^2\cdot$s or more. If air permeability is less than 300 cc/cm$^2\cdot$s, the stocking feels hot and sticky.

The moisture absorption rate of the compression stocking of the present embodiment in an environment at 35°C and 90% RH is preferably 7% or more, more preferably 9% or more and even more preferably 12% or more. If the moisture absorption rate is less than 7%, the sticky sensation felt when wearing cannot be adequate suppressed creating a feeling of discomfort.

There are no particular limitations on the method used to impart moisture absorption to the compression stocking of the present embodiment, and hygroscopic fibers or fibers incorporating a substance that allows the obtaining of moisture absorption may be used, or a processing agent that generates a moisture absorption effect may be imparted. An example of a preferred embodiment is the use of cellulose fiber. The content of the cellulose fiber is set so that the moisture absorption rate of the compression stocking is 7% or more and there are no particular limitations thereon. A blend of synthetic fiber and cellulose fiber can be used preferably. Although the blend ratio between the synthetic fiber and cellulose fiber can be set arbitrarily, it is preferably 20% to 80% and more preferably 25% to 75%. Although it is preferable to arrange the cellulose fiber in the foot portion since the soles and toes of the feet easily become hot and sticky, it is preferable to employ a two-layer structure for the soles of the feet to prevent yarn breakage due to abrasion. Cellulose fiber or a composite yarn thereof is arranged on the skin side while nylon-processed yarn is arranged on the outside. A cut boss structure is preferably used for the two-layer structure.

EXAMPLES

The following provides an explanation of the present invention using examples.

Each of the evaluation methods used in the examples are as indicated below.

(1) Stress when Calf Portion Stretched in Radial Direction to Worn State

A sample is collected from the calf portion of a compression stocking, the sample clamped at width of 2.5 cm and clamping interval of 5 cm is repeatedly stretched to the worn state and allowed to recover three times at a pulling speed of 300 mm/min using a tensile tester, and stress is measured during the first round of stretching.

The worn state refers to a state in which an elastic stocking is stretched to $((Y-2X)/2X) \times 100$ (%) when the dimension of the measured site of the elastic stocking (equivalent to the width of a folded product) is taken to be X cm and the circumference of the calf portion of a wooden model leg for measuring compression manufactured by Swiss Lastic of the corresponding size is taken to be Y cm.

(2) Stress when Ankle Portion Stretched in Radial Direction until Passes Heel

A sample is collected from the ankle portion of a compression stocking using the same method as in (1) above, and stress is measured when stretched to the maximum elongation rate when passing over the heel portion of a wooden model leg for measuring compression manufactured by Swiss Lastic of the corresponding size.

(3) Stress during Ankle Portion Elongation Rate of 10%

A sample is collected from the ankle portion of a compression stocking using the method as in (1) above, and stress is measured during the first time the sample was stretched at an elongation rate of 10%.

(4) Elongation Rate during Load of 22.1 N in Lengthwise Direction

A sample is collected from the ankle portion of a compression stocking, and clamping length is measured when knitted fabric clamped at a width of 2.5 cm and clamping interval of 5 cm is stretched at a pulling speed of 300 mm/min to a load of 22.1 N. N=3 samples are measured and elongation rate (%) is determined using the equation below.

$$\text{Elongation rate (\%)} = \{(\text{clamping length during stretching (cm)} - 5 \text{ (cm)})/5 \text{ cm}\} \times 100$$

(5) Compression

An air bag connected to the Model AMI-3037-10 Compression Analyzer manufactured by AMI Techno Co., Ltd. is attached to a prescribed site of a wooden model leg for measuring compression manufactured by Swiss Lastic followed by measurement of compression (hPa) when the compression stocking of the present embodiment is worn (first time worn). Following this measurement, the stocking is removed from the model leg and compression is measured when worn again (second time worn). The stocking is repeatedly put on and taken off ten times according to this procedure and compression is measured a total of ten times followed by determination of the average thereof. Size S corresponds to a circumference of the calf of a human body of 28 cm to 34 cm and a type 5 is used for the model leg, size M corresponds to a circumference of the calf of a human body of 32 cm to 38 cm and a type 7 is used for the model leg, and size L corresponds to a circumference of the calf of a human body of 36 cm to 42 cm and a type 10 is used for the model leg.

(6) Dynamic Friction Coefficient and Surface Roughness

The skin side of a sample in which the calf portion of the compression stocking of the present embodiment is stretched to the worn state of a wooden model leg for measuring compression manufactured by Swiss Lasitc of an appropriate size is measured in the lengthwise direction for dynamic friction coefficient, standard deviation of dynamic friction coefficient and surface roughness with the Model KES-FB4-AUTO-A Surface Tester manufactured by Kato Tech Co., Ltd. followed by determination of the respective average thereof.

(7) Thickness

A measuring probe having a diameter of 3.0 cm is contacted at a load of 5 g with the calf portion of a stocking cut open and in the state of a single piece of fabric followed by measurement of thickness at three locations using a thickness measurement gauge manufactured by Peacock Corp. and determination of the average thereof.

(8) Moisture Absorption Rate

After allowing a size M compression stocking of the present embodiment to stand for 4 hours in a hot air dryer at 105°C±2°C until it reaches a constant weight, the stocking is placed in a sealed polyethylene bag followed by allowing to cool by standing for 1 hour indoors at 20°C and weighing the absolute dry weight with an electronic balance. After weighing, the compression stocking of the present embodiment is taken out of the sealed polyethylene bag and allowed to stand for 15 minutes on a desk in an environment at 35°C and 90% RH followed by weighing again with an electronic balance. N=3 samples are measured and moisture absorption rate is determined from equation (D) below.

$$\text{Moisture absorption rate (\%)} = \{\text{weight (g) after standing for 15 minutes at 35°C and}$$
$$\text{90\% RH - absolute dry weight (g)}\}/\text{absolute dry weight} \times 100 \qquad \text{(D)}$$

(9) Air Permeability

Air permeability (cc/cm$^2$·sec) of the calf portion is measured for N=3 samples with a JIS Frazier air permeability tester followed by determination of the average thereof. In the case air permeability exceeds 400 cc/cm$^2$·sec and cannot be measured, air permeability may be measured using the Model KES Air Permeability Tester manufactured by Kato Tech Co., Ltd. and then converted.

(10) Yarn Length of Non-Elastic Yarn and Elastic Yarn

A range of 100 wales is marked out on a knitted fabric followed by unraveling the non-elastic yarn and elastic yarn from the knitted fabric. The upper end of the unraveled non-elastic yarn is immobilized and a load of 0.088 cN/dtex is applied to the lower end followed by measuring the length after 30 seconds. The upper end of the unraveled elastic yarn is immobilized and length is measured in the state in which the elastic yarn is elongated to a degree that is it is not stretched. The average is calculated for N=3 samples.

(11) Removability

The stocking was put on and taken off by five subjects followed by a subjective evaluation of removability according to the evaluation criteria indicated below. An evaluation of 3 or higher indicates that the stocking demonstrates an effect.

5: Hardly any force required to put on and take off the stocking and able to be put on and taken off easily.
4: Not very much force required to put on and take off the stocking and able to be put on and taken off relatively easily.
3: Some force required to put on and take off the stocking and able to be put on and taken off with ordinary ease.
2: Force required to put on and take off the stocking and time required to put on and take off.
1: Considerable force required to put on and take off the stocking and an extremely long time required to put on and take off.

(12) Hot and Sticky Sensation

The hot and sticky sensation felt when five subjects wore the stocking for 8 hours was subjectively evaluated according to the evaluation criteria indicated below. An evaluation of 3 or higher indicates that stocking demonstrates an effect.

5: Not hot and sticky sensation whatsoever
4: Hardly any hot and sticky sensation
3: Slight hot and sticky sensation
2: Considerable hot and sticky sensation
1: Extremely strong hot and sticky sensation

(13) Swelling Sensation

A swelling sensation felt when five subjects wore the stocking for 8 hours was subjectively evaluated according to the evaluation criteria indicated below. An evaluation of 3 or higher indicates that stocking demonstrates an effect.

5: No swelling sensation whatsoever
4: Hardly any swelling sensation
3: Slight swelling sensation
2: Considerable swelling sensation
1: Extremely strong swelling sensation

(14) Amount of Swelling

The amount of swelling when the stocking was worn by seven subjects for 8 hours was evaluated according to a change in the circumference of the calf between prior to wearing the stocking and immediately after taking off the stocking. Circumference was measured while not wearing the compression stocking for both before and after wearing.

$$\text{Amount of swelling } (\%) = (\text{circumference after wearing for 8 hours - circumference before wearing})/(\text{circumference before wearing}) \times 100$$

[EXAMPLE 1]

A knee-length compression stocking was knit according to the knitting structure shown in FIG. 2 using 235 dtex polyurethane elastic yarn, having a polyether group having two methyl groups as side chains on the same carbon atom for a portion of the polyether skeleton composing a diol component, for the elastic yarn for those portions other than the toes, heel portion and rubber top band, and using 66 dtex × 43f cupra and 36 dtex × 26f nylon for the non-elastic yarn, using the Model LA45ME panty stocking knitting machine manufactured by Lonati SPA having a kiln diameter of 4.5 inches and a needle count of 400. Non-elastic yarn was arranged for A and B and elastic yarn was arranged for C, and the amount of yarn supplied was suitable adjusted to obtain the compression shown in the following Table 1. The amount of yarn supplied in the ankle portion (yarn length (cm) per 100 wales (W)) is shown in Table 1. Compression was gradually changed between set portions. However, nylon-processed yarn having a plain stitch structure was used for the toes and heel, and non-elastic yarn and elastic yarn having a rib stitch structure were used for the rubber top band. The sole portions of the feet were reinforced by arranging nylon yarn on the surface of cupra-nylon composite yarn in a cut boss structure. Subsequently, the toes were sewn followed by presetting, dyeing and softening processing, after which the stocking was steam set to obtain a size M compression stocking having the properties described in the following Table 1. The resulting compression stocking had superior removability and was free of any hot and sticky sensation or swelling sensation.

[EXAMPLES 2 to 5]

Compression stockings having the properties described in Table 1 were obtained using the same method as Example 1 with the exception of changing the compression design. Evaluation results of the resulting compression stockings are shown in Table 1.

[EXAMPLE 6]

Knitting was carried out according to the knitting structure shown in FIG. 3 using the same knitting machine and using the same yarn as in Example 1. Non-elastic yarn was arranged for A and B and elastic yarn was arranged for C. Subsequently, bleaching processing and softening processing were carried out followed by steam setting to obtain a compression stocking having the properties described in the following Table 1. The resulting compression stocking demonstrated somewhat low dynamic frictional resistance, favorable removability, and was resistant to causing a hot and sticky sensation or swelling sensation.

[EXAMPLE 7]

A compression stocking having the properties described in the following Table 1 was obtained using the same method as Example 6 with the exception of changing the yarn length of the non-elastic yarn and elastic yarn. The resulting compression stocking was resistant to causing a hot and sticky sensation or swelling sensation.

[EXAMPLE 8]

Knitting was carried out according to the knitting structure shown in FIG. 4 using the same knitting machine and using the same yarn as in Example 1. Non-elastic yarn was arranged for A, B and D and elastic yarn was arranged for C. Subsequently, bleaching processing and softening processing were carried out followed by steam setting to obtain a compression stocking having the properties described in the following Table 1. The resulting compression stocking demonstrated favorable removability and was resistant to causing a hot and sticky sensation or swelling sensation.

[EXAMPLE 9]

Knitting was carried out according to the knitting structure shown in FIG. 2 using DCY obtained by changing to the polyurethane elastic yarn used in Example 1 and double covering nylon 33T around the same polyurethane elastic yarn followed by carrying out bleaching processing and softening processing and then steam setting to obtain a compression stocking having the properties described in the following Table 1. Although the resulting compression stocking demonstrated compression that was somewhat higher than that of Example 1 and dynamic frictional resistance was somewhat high, removability was favorable and the stocking did not cause a hot and sticky sensation or swelling sensation.

[EXAMPLE 10]

The properties of a compression stocking knit according to the knitting structure of FIG. 2 using 167 dtex/72 f nylon processed yarn for the non-elastic yarn of Example 1 are described in the following Table 1. The resulting compression stocking demonstrated favorable removability, and although caused a hot and sticky sensation, did not cause a swelling sensation.

[EXAMPLE 11]

Knitting was carried out to below the knee using the same knitting design as Example 1 followed by further knitting to the base of the thigh so that compression of the thigh portion reached the value shown in the following Table 1 and also knitting the panty portion. Nylon yarn was used for the panty portion and a compression stocking was obtained having the properties described in the following Table 1. Removability of the resulting compression panty stocking was favorable and there was no hot and sticky sensation or swelling sensation.

[COMPARATIVE EXAMPLE 1]

A knee-length compression stocking was knit according to the knitting structure shown in FIG. 5 using DCY, obtained by double covering 130 dtex polyurethane elastic yarn with 33 dtex/10f nylon yarn, as elastic yarn and using 56 dtex/5f two-ply nylon-processed yarn as non-elastic yarn for the portion covering from the ankle to the calf and from the ankle to the toes excluding the tips of the toes, heel portion and rubber top band, using the Model 45ME panty stocking knitting machine manufactured by Lonati SPA having a kiln diameter of 4.5 inches and a needle count of 400. Non-elastic yarn was arranged for A and elastic yarn was arranged for C. Properties of the resulting compression stocking are described in the following Table 1. Although the stocking was difficult to put on and take off and caused a hot and sticky sensation, there was no swelling sensation.

[COMPARATIVE EXAMPLE 2]

A compression stocking having the properties described in the following Table 1 was obtained by knitting according to the knitting structure of FIG. 6 by knitting together 78 dtex polyurethane elastic yarn, in which a portion of a diol component has two methyl groups as a side chain on the same carbon atom, as elastic yarn for the portion covering from the ankle to the calf and from the ankle to the toes excluding the tips of the toes, heel portion and rubber top band, and false twist composite yarn consisting of 66dtex $\times$ 43f cupra and 36 dtex $\times$ 26f nylon as non-elastic yarn, using the Model 45ME panty stocking knitting machine manufactured by Lonati SPA having a kiln diameter of 4.5 inches and a needle count of 400, followed by subjecting to bleaching processing and softening processing and then steam setting. The resulting compression stocking demonstrated a high constant load elongation rate in the lengthwise direction, was difficult to put on and take off, and did not cause a hot and sticky sensation or swelling sensation.

[COMPARATIVE EXAMPLE 3]

A compression stocking having the properties described in the following Table 1 was obtained using the same method as Example 1 with the exception of changing the yarn length of the non-elastic yarn and elastic yarn. The resulting compression stocking demonstrated extremely high compression, constant elongation stress of the ankle portion in the radial direction was high, the stocking was difficult to put on and take off, and although it caused somewhat of a hot and sticky sensation, it did not cause a swelling sensation.

[COMPARATIVE EXAMPLE 4]

A compression stocking having the properties described in the following Table 1 was obtained using the same method as Example 1 with the exception of changing the yarn length of the non-elastic yarn and elastic yarn. The resulting compression stocking demonstrated low compression, constant elongation stress of the ankle portion in the radial direction was low, and although the stocking was easy to put on and take off, it caused a strong hot and sticky sensation and swelling sensation.

[Table 1]

| | | Ex.1 | Ex.2 | Ex.3 | Ex.4 | Ex.5 | Ex.6 | Ex.7 | Ex.8 | Ex.9 | Ex.10 | Ex.11 | Comp Ex. 1 | Comp. Ex. 2 | Comp. Ex. 3 | Comp. Ex. 4 |
|---|---|---|---|---|---|---|---|---|---|---|---|---|---|---|---|---|
| Leg yarn and knitting structure | Non-elastic yarn | BB66/NY36 | ← | ← | ← | ← | ← | ← | ← | ← | NY167 | BB66/NY36 | NY56 | BB66/NY36 | BB66/NY36 | BB66/NY36 |
| | Elastic yarn | PU235 | ← | ← | ← | ← | ← | ← | ← | DCY | PU235 | PU235 | DCY | PU78 | DCY | PU235 |
| | Structure | FIG.2 | FIG. 2 | FIG. 2 | FIG. 2 | FIG. 2 | FIG. 3 | FIG. 3 | FIG. 4 | FIG. 2 | FIG. 2 | FIG. 2 | FIG. 5 | FIG. 6 | FIG. 2 | FIG. 2 |
| Stress in radial direction of calf portion when worn | | 2.5 | 2.1 | 5.8 | 3.5 | 2.9 | 2.4 | 3.6 | 3.9 | 3.1 | 2.4 | 2.5 | 7.1 | 3.9 | 7.5 | 0.85 |
| Stress in radial direction of ankle portion when passed over heel | | 4.2 | 3.7 | 6.8 | 4.1 | 3.5 | 3.9 | 4 | 6.5 | 4.6 | 4 | 4.3 | 5.4 | 7.3 | 7.8 | 1.5 |
| 10% elongation stress of ankle portion in radial direction | | 0.41 | 0.22 | 0.65 | 0.4 | 0.34 | 0.35 | 0.37 | 0.69 | 0.72 | 0.38 | 0.41 | 1.15 | 0.48 | 0.83 | 0.13 |
| Elongation rate during load of 22.1 N in vertical direction | Ankle | 69 | 72 | 52 | 65 | 60 | 80 | 33 | 48 | 63 | 72 | 69 | 60 | 110 | 40 | 48 |
| Standard deviation of dynamic friction coefficient | | 0.018 | 0.025 | 0.017 | 0.016 | 0.02 | 0.043 | 0.041 | 0.035 | 0.069 | 0.017 | 0.018 | 0.133 | 0.103 | 0.015 | 0.033 |
| Mean deviation of surface roughness μm | | 4.5 | 4.8 | 4 | 4.6 | 4.6 | 4.2 | 4.1 | 4.9 | 5.4 | 4.3 | 4.1 | 11.0 | 10.3 | 5.5 | 6.8 |
| Compression (hPa) | Instep | 10 | 15 | 15 | 15 | 34 | 15 | 20 | 25 | 18 | 12 | 28 | 20 | 25 | 35 | 10 |
| | Ankle | 27 | 20 | 42 | 27 | 24 | 29 | 30 | 45 | 32 | 26 | 27 | 42 | 35 | 55 | 21 |
| | Lower calf | 24 | 17 | 38 | 36 | 30 | 24 | 38 | 34 | 26 | 24 | 25 | 35 | 25 | 51 | 12 |
| | Calf | 22 | 16 | 41 | 31 | 30 | 24 | 33 | 36 | 26 | 22 | 23 | 35 | 25 | 51 | 10 |
| | Thigh | | | | | | | | | | | | 12 | | | |
| Thickness (mm) | | 0.64 | 0.67 | 0.62 | 0.65 | 0.66 | 0.62 | 0.63 | 0.7 | 0.78 | 0.61 | 0.63 | 0.44 | 0.74 | 0.71 | 0.6 |

EP 3 632 245 B1

13

(continued)

| | | Ex.1 | Ex.2 | Ex.3 | Ex.4 | Ex.5 | Ex.6 | Ex.7 | Ex.8 | Ex.9 | Ex.10 | Ex.11 | Comp Ex. 1 | Comp. Ex. 2 | Comp. Ex. 3 | Comp. Ex. 4 |
|---|---|---|---|---|---|---|---|---|---|---|---|---|---|---|---|---|
| Length of non-elastic yarn A (cm/100 W) | | 15.6 | 17.1 | 14.2 | 15.7 | 15.9 | 17.8 | 18.1 | 14.8 | 15.2 | 15.8 | 15.8 | 12.2 | | 13.8 | 18.1 |
| Length of non-elastic yarn B (cm/100 W) | | 24.4 | 27.8 | 22.2 | 24.6 | 25.5 | 21.5 | 21.4 | 29.5 | 24.2 | 24.8 | 24.6 | | 18.5 | 21.3 | 28.8 |
| Length of non-elastic yarn D (cm/100 W) | | | | | | | | | 10.1 | | | | | | | |
| Length of elastic yarn C (cm/100 W) | | 4.4 | 4.8 | 4.2 | 4.4 | 4.5 | 4.4 | 4.3 | 4.1 | 4.6 | 4.5 | 4.5 | 4.2 | 4.1 | 3.7 | 5.1 |
| Air permeability (cc/cm$^2$/sec) | | 1700 | 2140 | 1225 | 1460 | 1440 | 1720 | 1550 | 1330 | 1770 | 1150 | 1660 | 260 | 1480 | 630 | 2480 |
| Moisture absorption rate (%) | | 17.4 | 16.9 | 18.1 | 17.4 | 17.1 | 15.5 | 15.3 | 12.2 | 16.1 | 6.5 | 17.6 | 6.4 | 18.2 | 15.1 | 16.6 |
| Elastic yarn surface exposure rate % | | 26.5 | 28.8 | 24.5 | 26.2 | 27 | 18.8 | 16.6 | 32.8 | 5.8 | 26.9 | 27.1 | 6.8 | 30.5 | 5.2 | 31.5 |
| Removability | | 4.6 | 5 | 3.6 | 4.6 | 5 | 4.8 | 4.4 | 3.2 | 3.8 | 4.6 | 4.6 | 1.2 | 1.8 | 2.2 | 5 |
| Hot and sticky sensation | | 4.8 | 4.8 | 4.6 | 4.8 | 4.8 | 4.8 | 4.8 | 4.6 | 4.8 | 1.8 | 4.8 | 1.4 | 4.8 | 4.8 | 4.6 |
| Swelling sensation | | 4.6 | 3.4 | 4.8 | 5 | 5 | 4.6 | 4.8 | 4.8 | 4.4 | 4.4 | 4.6 | 4.4 | 3.6 | 4.8 | 1.2 |
| Swelling % | | 0.1 | 1.1 | -0.2 | -0.4 | -0.5 | 0.2 | -0.2 | -0.2 | 0.1 | 0 | 0.1 | -0.2 | 0.3 | -0.5 | 1.5 |

INDUSTRIAL APPLICABILITY

Use of the compression stocking of the present invention makes it possible to provide a stocking, which together with demonstrating preventive effects against routine swelling and venous edematous disorders, has easy removability.

REFERENCE SIGNS LIST

a   Instep
b   Ankle portion
c   Lower calf portion
d   Calf portion
e   Thigh portion

**Claims**

1.   A compression stocking that at least covers an ankle portion (b), a lower calf portion (c) and a calf portion (d), wherein the stress when the calf portion (d) is stretched in the radial direction to a worn state, determined as described in the description, is 1 N to 7 N, the stress when the ankle portion (b) is stretched in the radial direction until it passes over the heel, determined as described in the description, is 1 N to 7 N, and initial load when the ankle portion (b) is stretched in the radial direction to an elongation rate of 10% is 1 N or less, **characterized in that** the elongation rate of the ankle portion (b) in the lengthwise direction during a load of 22.1 N, determined as described in the description, is 10 % to 100 %.

2.   The compression stocking according to claim 1, wherein the compression stocking is composed by circular knitting.

3.   The compression stocking according to claim 1 or 2, wherein an elastic yarn is insert-knitted into a portion at least containing the ankle portion (b), lower calf portion (c) and calf portion (d) of the compression stocking.

4.   The compression stocking according to claim 3, wherein the elastic yarn is insert-knitted by using bare yarn.

5.   The compression stocking according to any of claims 1 to 4, wherein the compression of the calf portion, determined as described in the description, is 15 hPa to 50 hPa.

6.   The compression stocking according to claim 5, wherein the compression of the lower calf portion (c) is 3 hPa to 15 hPa greater than the compression of the calf portion (d).

7.   The compression stocking according to claim 5 or 6, wherein the compression of the instep portion (a) is 3 hPa to 15 hPa greater than the compression of the calf portion (d).

8.   The compression stocking according to any of claims 1 to 7, wherein the elongation rate of the ankle portion (b) in the lengthwise direction during a load of 22.1 N is 10% to 70%.

9.   The compression stocking according to any of claims 1 to 8, wherein the standard deviation of the dynamic friction coefficient on the skin side of the ankle portion (b) is 0.005 to 0.08.

10.   The compression stocking according to any of claims 5 to 9, wherein the compression of the ankle portion (b) is 5 hPa to 35 hPa and smaller than the compression of the calf portion (d).

11.   The compression stocking according to any of claims 1 to 10, wherein the air permeability of the calf portion is 150 cm$^3$fcm$^2$·s or more.

12.   The compression stocking according to any of claims 1 to 11, wherein the moisture absorption rate, determined as described in the description, is 7% or more.

**Patentansprüche**

1. Kompressionsstrumpf, der wenigstens einen Knöchelteil (b), einen unteren Wadenteil (c) und einen Wadenteil (d) bedeckt, wobei die Spannung, wenn der Wadenteil (d) in radialer Richtung bis zu einem verbrauchten Zustand gedehnt wird, welche so bestimmt wird, wie es in der Beschreibung beschrieben ist, 1 N bis 7 N beträgt, die Spannung, wenn der Knöchelteil (b) in radialer Richtung gedehnt wird, bis er sich über die Ferse bewegt, welche so bestimmt wird, wie es in der Beschreibung beschrieben ist, 1 N bis 7 N beträgt, und die Anfangsbelastung, wenn der Knöchelteil (b) in radialer Richtung bis zu einer Dehnung von 10% gedehnt wird, 1 N oder weniger beträgt, **dadurch gekennzeichnet, dass** die Dehnung des Knöchelteils (b) in Längsrichtung während einer Belastung von 22,1 N, welche so bestimmt wird, wie es in der Beschreibung beschrieben ist, 10% bis 100% beträgt.

2. Kompressionsstrumpf gemäß Anspruch 1, wobei der Kompressionsstrumpf durch Rundstricken zusammengesetzt wird.

3. Kompressionsstrumpf gemäß Anspruch 1 oder 2, wobei in einen Teil, der wenigstens den Knöchelteil (b), den unteren Wadenteil (c) und den Wadenteil (d) des Kompressionsstrumpfs enthält, ein elastisches Garn mit eingestrickt wird.

4. Kompressionsstrumpf gemäß Anspruch 3, wobei das elastische Garn unter Verwendung von bloßem Garn mit eingestrickt wird.

5. Kompressionsstrumpf gemäß einem der Ansprüche 1 bis 4, wobei die Kompression des Wadenteils, die so bestimmt wird, wie es in der Beschreibung beschrieben ist, 15 hPa bis 50 hPa beträgt.

6. Kompressionsstrumpf gemäß Anspruch 5, wobei die Kompression des unteren Wadenteils (c) um 3 hPa bis 15 hPa größer ist als die Kompression des Wadenteils (d).

7. Kompressionsstrumpf gemäß Anspruch 5 oder 6, wobei die Kompression des Ristbereichs (a) um 3 hPa bis 15 hPa größer ist als die Kompression des Wadenteils (d).

8. Kompressionsstrumpf gemäß einem der Ansprüche 1 bis 7, wobei die Dehnung des Knöchelteils (b) in Längsrichtung während einer Belastung von 22,1 N 10% bis 70% beträgt.

9. Kompressionsstrumpf gemäß einem der Ansprüche 1 bis 8, wobei die Standardabweichung des dynamischen Reibungskoeffizienten auf der Hautseite des Knöchelteils (b) 0,005 bis 0,08 beträgt.

10. Kompressionsstrumpf gemäß einem der Ansprüche 5 bis 9, wobei die Kompression des Knöchelteils (b) 5 hPa bis 35 hPa beträgt und kleiner ist als die Kompression des Wadenteils (d).

11. Kompressionsstrumpf gemäß einem der Ansprüche 1 bis 10, wobei die Luftdurchlässigkeit des Wadenteils 150 cm$^3$fcm$^2$·s oder mehr beträgt.

12. Kompressionsstrumpf gemäß einem der Ansprüche 1 bis 11, wobei die Feuchtigkeitsaufnahme, die so bestimmt wird, wie es in der Beschreibung beschrieben ist, 7% oder mehr beträgt.

**Revendications**

1. Bas de contention qui recouvre au moins une portion de cheville (b), une portion de mollet inférieur (c) et une portion de mollet (d), dans lequel la contrainte lorsque la portion de mollet (d) est étirée dans la direction radiale jusqu'à un état usé, déterminée comme décrit dans la description, est de 1 N à 7 N, la contrainte lorsque la portion de cheville (b) est étirée dans la direction radiale jusqu'à ce qu'elle passe sur le talon, déterminée comme décrit dans la description, est de 1 N à 7 N, et la charge initiale lorsque la portion de cheville (b) est étirée dans la direction radiale à un taux d'allongement de 10 % est de 1 N ou inférieure, **caractérisé en ce que** le taux d'allongement de la portion de cheville (b) dans la direction longitudinale pendant une charge de 22,1 N, déterminé comme décrit dans la description, est de 10 % à 100 %.

2. Bas de contention selon la revendication 1, dans lequel le bas de contention est composé par tricotage circulaire.

3. Bas de contention selon la revendication 1 ou 2, dans lequel un fil élastique est inséré-tricoté dans une portion contenant au moins les portions de cheville (b), portion de mollet inférieure (c) et portion de mollet (d) du bas de contention.

4. Bas de contention selon la revendication 3, dans lequel le fil élastique est inséré-tricoté en utilisant du fil nu.

5. Bas de contention selon l'une quelconque des revendications 1 à 4, dans lequel la contention de la portion de mollet, déterminée comme décrit dans la description, est de 15 hPa à 50 hPa.

6. Bas de contention selon la revendication 5, dans lequel la contention de la portion de mollet inférieur (c) est de 3 hPa à 15 hPa supérieure à la contention de la portion de mollet (d).

7. Bas de contention selon la revendication 5 ou 6, dans lequel la contention de la portion de coup de pied (a) est de 3 hPa à 15 hPa supérieure à la contention de la portion de mollet (d).

8. Bas de contention selon l'une quelconque des revendications 1 à 7, dans lequel le taux d'allongement de la portion de cheville (b) dans la direction longitudinale pendant une charge de 22,1 N est de 10 % à 70 %.

9. Bas de contention selon l'une quelconque des revendications 1 à 8, dans lequel l'écart type du coefficient de friction dynamique sur le côté de la peau de la portion de cheville (b) est de 0,005 à 0,08.

10. Bas de contention selon l'une quelconque des revendications 5 à 9, dans lequel la contention de la portion de cheville (b) est de 5 hPa à 35 hPa et inférieure à la contention de la portion de mollet (d).

11. Bas de contention selon l'une quelconque des revendications 1 à 10, dans lequel la perméabilité à l'air de la portion de mollet est de 150 $cm^3/cm^2$.s ou supérieure.

12. Bas de contention selon l'une quelconque des revendications 1 à 11, dans lequel le taux d'absorption d'humidité, déterminé comme décrit dans la description, est de 7 % ou supérieur.

# FIG. 1

# FIG. 2

|   | 1 | 2 | 3 | 4 |
|---|---|---|---|---|
| A | K | W | K | W |
| C | T | W | T | W |
| B | K | K | K | K |

# FIG. 3

|   | 1 | 2 | 3 | 4 |
|---|---|---|---|---|
| B | K | K | K | K |
| C | T | W | T | W |
| A | W | K | W | K |

## FIG. 4

|   | 1 | 2 | 3 | 4 |
|---|---|---|---|---|
| A | W | K | W | K |
| B | K | K | K | K |
| C | T | W | T | W |
| D | W | K | W | K |

## FIG. 5

|   | 1 | 2 | 3 | 4 |
|---|---|---|---|---|
| A | K | W | K | W |
| C | T | W | T | W |
| A | W | K | W | K |
| C | W | T | W | T |

## FIG. 6

|      | 1 | 2 | 3 | 4 |
|------|---|---|---|---|
| B、C | K | K | K | K |

FIG. 7

|   | 1 | 2 | 3 | 4 |
|---|---|---|---|---|
| B | K | K | K | K |
| C | T | W | T | W |
| A | K | W | K | W |

**EP 3 632 245 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030213269 A1
- JP 2005240222 A
- JP 2010077574 A